# EUROPEAN PATENT APPLICATION

(11) **EP 2 849 211 A1**
(43) Date of publication of application: **18.03.2015**
(21) Application number: 13787729.6
(22) Date of filing: 10.05.2013
(51) Int. Cl.: H01L 21/308, H01L 31/04

(54) **ETCHING FLUID AND PRODUCTION METHOD FOR SILICON-BASED SUBSTRATE USING SAME**

(30) Priority: 11.05.2012 JP 2012108967
(71) Applicant: Wako Pure Chemical Industries, Ltd., Osaka-shi Osaka 540-8605 (JP)
(72) Inventor: OHUCHI Naoko, Kawagoe-shi Saitama 350-1101 (JP); KAKIZAWA Masahiko, Kawagoe-shi Saitama 350-1101 (JP); TSURUMOTO Hiroyuki, Kawagoe-shi Saitama 350-1101 (JP); WATANABE Terumi, Kawagoe-shi Saitama 350-1101 (JP); KAWARA Shinshi, Kawagoe-shi Saitama 350-1101 (JP)
(74) Representative: Hinkelmann, Klaus
(86) International application number: PCT/JP2013/063222
(87) International publication number: WO 2013/168813

(57) **Abstract**

It is the object of the present invention to provide an alkali etching solution for solar cell manufacturing, which is capable of forming uniformly a fine hubbly structure throughout a whole wafer on the surface of a wafer having a silicon as a main component, and still more is applicable to various wafers; and a method for manufacturing a silicon-based substrate for solar cell manufacturing, using the etching solution. The present invention relates to the alkali etching solution for solar cell manufacturing, comprising (A) a mono- or disulfonic acid or a salt thereof represented by the general formula [1], (B) an alkali compound, and (C) water; and a method for manufacturing a silicon-based substrate for solar cell manufacturing, characterized by etching a wafer having a silicon as a main component, using the etching solution, to form a hubbly structure at the surface of the wafer: 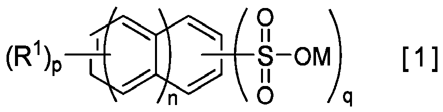 {wherein p moieties of R¹ each independently represents a hydrogen atom, a hydroxyl group or an alkyl group having 1 to 10 carbon atoms; q moieties of M each independently represents a hydrogen atom, an alkali metal atom, an ammonium (NH₄) group or a tetraalkylammonium (R²₄N) group (wherein R² represents an alkyl group having 1 to 4 carbon atoms); n represents 0 or 1; and p and q each independently represents 1 or 2}.

## Description

### TECHNICAL FIELD

The present invention relates to the alkali etching solution for solar cell manufacturing which is useful as a semiconductor substrate for a solar cell, and is suitable for manufacturing a silicon-based substrate having a silicon as a main component and having a hubbly structure at the surface; and a method for manufacturing a silicon-based substrate for solar cell manufacturing, which comprises etching a wafer having a silicon as a main component, by using the etching solution, to form a hubbly structure at the surface of the wafer.

### BACKGROUND ART

Recently, under background of environmental problems, a solar cell, which is one of the clean energy, has attracted attention and demand thereof has been increased. However, energy manufacturing cost of a solar cell is higher as compared with general commercial power, which is a reason for inhibiting the spread thereof. In order to reduce cost of a solar cell, which is such an obstacle to the spread, it is said necessary to enhance conversion efficiency (photoelectric conversion efficiency) thereof, while to reduce manufacturing cost of a substrate. The conversion efficiency (photoelectric conversion efficiency) is a value represented by ratio of energy which is capable of taking out by converting the light energy to the electric energy, in percent, and it is the most important characteristics as a solar cell. As a solar cell having this high conversion efficiency (photoelectric conversion efficiency), there is included a compound semiconductor solar cell, where the conversion efficiency has reached near 25%, however, as for the compound semiconductor solar cell, it is extremely difficult to prepare the compound semiconductor to be used as a material, which has a problem of general prevalence in view of manufacturing cost, and thus applications thereof are limited. Accordingly, although it is inferior to the compound semiconductor solar cell, in view of the conversion efficiency (photoelectric conversion efficiency), in view of easy procurement of a substrate and low manufacturing cost thereof, a monocrystal silicon solar cell having the conversion efficiency (photoelectric conversion efficiency) of around 20%, or a polycrystalline silicon solar cell having the conversion efficiency (photoelectric conversion efficiency) of about 5 to 15%, or the like has been a major source of generally prevailed solar cell.

In manufacturing such a monocrystal silicon solar cell, or a polycrystalline silicon solar cell, it is necessary to enhance the conversion efficiency thereof, by subjecting the surface of a silicon wafer to fine uneven processing, to form a texture structure by fine unevenness, and by repeated reflection of solar rays irradiated to the surface thereof at the uneven portion, to increase Jsc (short-circuit current density) characteristics.

Therefore, various technological developments for applying a fine uneven processing on the surface of a silicon wafer have been promoted. As such technologies, there have been known, for example, a technology for forming a hubbly structure on the silicon wafer surface by performing anisotropic etching using an aqueous solution containing sodium hydroxide and isopropyl alcohol (for example, PATENT LITERATURE 1, NON PATENT LITERATURE 1, or the like); a technology for performing a wet-type etching of a silicon monocrystal substrate using an aqueous solution containing 30% by weight or higher potassium hydroxide and an anionic surfactant (for example, PATENT LITERATURE 2, or the like); a technology for forming a hubbly structure on the semiconductor substrate surface using the alkali etching solution containing a carboxylic acid and silicon (for example, PATENT LITERATURE 3, or the like); a technology for forming a pyramid-shape unevenness at the silicon substrate surface using the etching solution containing an alkylsulfonic acid or the like and an alkali hydroxide in a concentration of 0.1% by weight to 30% by weight (for example, PATENT LITERATURE 4, or the like); and the like.

### CITATION LIST

### PATENT LITERATURE

PATENT LITERATURE 1: JP-A-61-96772
PATENT LITERATURE 2: JP-A-64-42824
PATENT LITERATURE 3: WO 2007/129555
PATENT LITERATURE 4: JP-A-2012-4528

### NON PATENT LITERATURE

NON PATENT LITERATURE 1: Progress in Photovoltaics: Research and Applications, Vol. 4, 435-438 (1996)

### SUMMARY OF INVENTION

### TECHNICAL PROBLEM

Thus, the etching technology of a silicon wafer (a semiconductor substrate) by adding various additives into an alkali aqueous solution has been known, however, the above-described technology has the following problems. That is, in the technology of PATENT LITERATURE 1 or NON PATENT LITERATURE 1, a composition ratio of the etching solution changes in etching, caused by high volatility of isopropyl alcohol, which results in irregularity in shape or size of a hubbly structure formed on the wafer surface, and thus a uniform hubbly structure cannot be formed throughout the whole wafer. In addition, in the technology of PATENT LITERATURE 2, the concentration of potassium hydroxide in the etching solution is too high, which not only inhibits to form a uniform hubbly structure but also makes application to a solar cell substrate unsuitable, due to high etching rate which provides too thin wafer after etching. Still more, the technology of PATENT LITERATURE 3 provides difficulty in forming a uniform hubbly structure, unless etching amount (matching allowance) is set high, which may provide the case of making a large difference in size of the hubbly structure. Still further, the technology of PATENT LITERATURE 4 provides fierce foaming of the etching solution in etching, due to inferior breaking of foam adhered at the wafer surface, when the wafer is pulled up, resulting in residual foam at the wafer surface, which tends to cause poor appearance of the wafer by generation of a watermark.

The present invention has been proposed in view of the above-described circumstance, and it is an object to provide tan alkali etching solution for solar cell manufacturing, which is capable of forming a fine hubbly structure throughout a whole wafer on the surface of a wafer having a silicon as a main component, and still more is applicable to various wafers; and a method for manufacturing a silicon-based substrate for solar cell manufacturing using the etching solution. The present inventors have intensively studied to develop such the alkali etching solution for solar cell manufacturing, and as a result, discovered that the etching solution, which has solved the above-described problems, can be provided, by adding a sulfonic acid having a specific structure to an alkali aqueous solution, and have thus completed the present invention.

### SOLUTION TO PROBLEM

The present invention consists of the following:
(1) The alkali etching solution for solar cell manufacturing, comprising (A) a mono- or disulfonic acid or a salt thereof represented by the general formula [1], (B) an alkali compound, and (C) water: 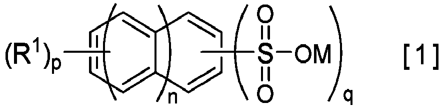 {wherein p moieties of R¹ each independently represents a hydrogen atom, a hydroxyl group or an alkyl group having 1 to 10 carbon atoms; q moieties of M each independently represents a hydrogen atom, an alkali metal atom, an ammonium (NH₄) group or a tetraalkylammonium (R²₄N) group (wherein R² represents an alkyl group having 1 to 4 carbon atoms); n represents 0 or 1; and p and q each independently represents 1 or 2}.
(2) A method for manufacturing a silicon-based substrate for solar cell manufacturing, which comprises etching a wafer having a silicon as a main component, by using the etching solution of the above-described (1), to form a hubbly structure at the surface of the wafer.

### ADVANTAGEOUS EFFECTS OF INVENTION

The alkali etching solution for solar cell manufacturing of the present invention is an etching solution which is capable of forming a pyramid-shape (quadrangular pyramid-shape) hubbly structure at the surface of a wafer having a silicon as a main component, and as compared with the conventional alkali etching solution, exerts an effect such as (1) being capable of forming a finer (smaller pyramid-shape) hubbly structure throughout a whole wafer surface, and generating almost no flat part, (2) being capable of forming a uniform hubbly structure throughout a whole wafer, without forming almost no inferior hubbly structure, (3) providing less foaming, and good breaking of foam adhered at a wafer surface, when the wafer is pulled up, thus resulting in foam disappearance from the wafer surface in a short time, and little generation of poor appearance of the wafer (watermark or the like) caused by remaining of foam at the wafer surface. Therefore, the alkali etching solution for solar cell manufacturing of the present invention can make effectively a substrate having high optical confinement effect (high light absorption rate and low light reflection rate) suitable for a solar cell. In addition, according to the alkali etching solution for solar cell manufacturing of the present invention, a pyramid-shape (quadrangular pyramid-shape) hubbly structure can be formed effectively, without generating manufacturing defect, even for a wafer prepared by a fixed abrasive grain method or mirror wafer, which has been said difficult to form a uniform hubbly structure throughout the whole wafer by a conventional method.

A manufacturing method of the silicon-based substrate for solar cell manufacturing of the present invention is an effective method for forming a hubbly structure on the surface of a wafer having a silicon as a main component, and by using the etching solution of the present invention, a pyramid-shape (quadrangular pyramid-shape) hubbly structure can be formed throughout a whole wafer on the surface of a wafer, and still more, a fine hubbly structure, which has the maximum side length of the bottom face of the pyramid is 0.1 µm to 25 µm, can be formed.

### BRIEF DESCRIPTION OF DRAWINGS

Figure 1 is a laser microscope photograph (magnification of 3000 times) of a wafer surface after etching obtained in Example 1.
Figure 2 is a laser microscope photograph (magnification of 3000 times) of a wafer surface after etching obtained in Comparative Example 1.
Figure 3 is a laser microscope photograph (magnification of 3000 times) of a wafer surface after etching obtained in Example 7.
Figure 4 is a laser microscope photograph (magnification of 3000 times) of a wafer surface after etching obtained in Example 9.

### DESCRIPTION OF EMBODIMENTS

The alkali etching solution for solar cell manufacturing of the present invention is the one characterized by comprising (A) a mono- or disulfonic acid or a salt thereof represented by the general formula [1], (B) an alkali compound, and (C) water: 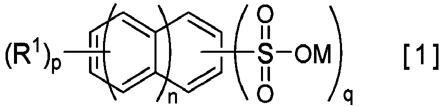 {wherein p moieties of R¹ each independently represents a hydrogen atom, a hydroxyl group or an alkyl group having 1 to 10 carbon atoms; q moieties of M each independently represents a hydrogen atom, an alkali metal atom, an ammonium (NH₄) group or a tetraalkylammonium (R²₄N) group (wherein R² represents an alkyl group having 1 to 4 carbon atoms); n represents 0 or 1; and p and q each independently represents 1 or 2}.

The alkyl group having 1 to 10 carbon atoms represented by R¹ in the general formula [1] includes a linear, branched or cyclic one, and specifically, for example, a methyl group, an ethyl group, a n-propyl group, an isopropyl group, a n-butyl group, an isobutyl group, a s-butyl group, a t-butyl group, a cyclobutyl group, a n-pentyl group, an isopentyl group, a s-pentyl group, a t-pentyl group, a neopentyl group, a 2-methylbutyl group, a 1,2-dimethylpropyl group, an 1-ethylpropyl group, a cyclopentyl group, a n-hexyl group, an isohexyl group, a s-hexyl group, a t-hexyl group, a neohexyl group, a 2-methylpentyl group, a 1,2-dimethylbutyl group, a 2,3-dimethylbutyl group, an 1-ethylbutyl group, a cyclohexyl group, a n-heptyl group, an isoheptyl group, a s-heptyl group, a t-heptyl group, a neoheptyl group, a cycloheptyl group, a n-octyl group, an isooctyl group, a s-octyl group, a t-octyl group, a neooctyl group, an 2-ethylhexyl group, a cyclooctyl group, a n-nonyl group, an isononyl group, a s-nonyl group, a t-nonyl group, a neononyl group, a cyclononyl group, a n-decyl group, an isodecyl group, a s-decyl group, a t-decyl group, a neodecyl group, a cyclodecyl group, a norbornyl group, a bornyl group, a menthyl group, an adamantyl group, a decahydronaphthyl group, or the like. Among these alkyl groups having 1 to 10 carbon atoms, for example, a linear, branched or cyclic alkyl group having 1 to 5 carbon atoms, such as a methyl group, an ethyl group, a n-propyl group, an isopropyl group, a n-butyl group, an isobutyl group, a s-butyl group, a t-butyl group, a cyclobutyl group, a n-pentyl group, an isopentyl group, a s-pentyl group, a t-pentyl group, a neopentyl group, a 2-methylbutyl group, a 1,2-dimethylpropyl group, an 1-ethylpropyl group, a cyclopentyl group is preferable, and among them, a methyl group, an ethyl group, a n-propyl group, or an isopropyl group, which is a linear or branched alkyl group having 1 to 3 carbon atoms, is more preferable, and further among them, a methyl group, which is the alkyl group having 1 carbon atom, is particularly preferable. It should be noted that, in the specific examples described above, n- represents normal-form, s- represents sec-form, and t- represents tert-form.

As R¹ in the general formula [1], a hydrogen atom or an alkyl group having 1 to 10 carbon atoms is more preferable, and among them, the alkyl group having 1 to 10 carbon atoms is particularly preferable.

In the case that the mono- or disulfonic acid relevant to the present invention is the monosulfonic acid (monosulfonic acid or salt thereof), when a carbon atom on a benzene ring bound with a sulfonic acid group is specified as 1 position, at least one moiety of p moieties of R¹ in the general formula [1] is preferably the one binding to a carbon atom at 2 position, 4 position or 6 position on the benzene ring, and among them, the one binding at 4 position is more preferable.

The alkali metal atom represented by M in the general formula [1] includes, for example, a sodium atom, a potassium atom, a lithium atom, or the like, and among them, a sodium atom, and a potassium atom are preferable.

The alkyl group having 1 to 4 carbon atoms represented by R², in the tetraalkyl ammonium group represented by an R²₄ N group in M of the general formula [1] includes a linear, branched or cyclic one, and specifically, for example, a methyl group, an ethyl group, n-propyl group, an isopropyl group, a n-butyl group, an isobutyl group, a s-butyl group, a t-butyl group, a cyclobutyl group, or the like, and among them, a methyl group which is an alkyl group having 1 carbon atom is preferable. It should be noted that, in the specific examples described above, n- represents normal-form, s- represents sec-form, and t- represents tert-form.

A specific example of the tetraalkyl ammonium group represented by R²₄N group in M of the general formula [1] includes, for example, a tetramethyl ammonium group, a tetraethyl ammonium group, a tetra-n-propyl ammonium group, a tetraisopropyl ammonium group, a tetra-n-butyl ammonium group, a tetraisobutyl ammonium group, a tetra-s-butyl ammonium group, a tetra-t-butyl ammonium group, a tetracyclobutyl ammonium group, or the like, and among them, a tetramethyl ammonium group is preferable. It should be noted that, in the specific examples described above, n- represents normal-form, s- represents sec-form, and t- represents tert-form.

As M in the general formula [1], a hydrogen atom is more preferable.

As n in the general formula [1], it is usually 0 or 1, and 0 is preferable.

As p in the general formula [1], it is usually 1 or 2, and 1 is preferable.

As q in the general formula [1], it is usually 1 or 2, and 1 is preferable.

A preferable example of the mono- or disulfonic acid relevant to the present invention in the alkali etching solution for solar cell manufacturing of the present invention include, for example, a mono- or disulfonic acid or a salt thereof represented by the following general formula [1'], and a mono- or disulfonic acid or a salt thereof represented by the following general formula [1 "], or the like. More specifically, the mono- or disulfonic acid or the salt thereof represented by the general formula [1'] represents a mono- or disulfonic acid or a salt thereof (benzene mono- or disulfonic acid derivative) when n in the general formula [1] is 0; and the mono- or disulfonic acid represented by the general formula [1 "], or a salt thereof represents a mono- or disulfonic acid, or a salt thereof (naphthalene mono or disulfonic acid derivative) when n in the general formula [1] is 1. 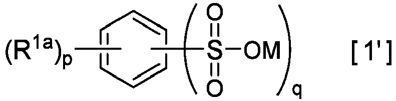 (wherein p moieties of R^{1a} each independently represents a hydrogen atom, a hydroxyl group or an alkyl group having 1 to 10 carbon atoms; and M, p and q are the same as described above.) 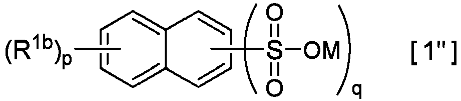 (wherein p moieties of R^{1b} each independently represents a hydrogen atom, a hydroxyl group or an alkyl group having 1 to 5 carbon atoms: and M, p and q are the same as described above.)

A specific example of the alkyl group having 1 to 10 carbon atoms represented by R^{1a} in the general formula [1'] includes the same one as those enumerated as the specific example of R¹ in the above-described general formula [1].

In the case where the mono- or disulfonic acid or the salt thereof represented by the general formula [1'] is the monosulfonic acid (monosulfonic acid or salt thereof), when a carbon atom on a benzene ring bound with a sulfonic acid group is specified as 1 position, at least one moiety among p moieties of R^{1a} in the general formula [1'] is preferably the one binding to a carbon atom at 2 position or 6 position (o-position) or 4 position (p-position) on the benzene ring, and among them, the one binding at 4 position (p-position) is more preferable.

The alkyl group having 1 to 5 carbon atoms represented by R^{1b} in the general formula [1 "], includes a linear, branched or cyclic one, and specifically, for example, a methyl group, an ethyl group, a n-propyl group, an isopropyl group, a n-butyl group, an isobutyl group, a s-butyl group, a t-butyl group, a cyclobutyl group, a n-pentyl group, an isopentyl group, a s-pentyl group, a t-pentyl group, a neopentyl group, a 2-methylbutyl group, a 1,2-dimethylpropyl group, an 1-ethylpropyl group, a cyclopentyl group, or the like, and among them, a methyl group, an ethyl group, a n-propyl group, or an isopropyl group, which is a linear or branched alkyl group having 1 to 3 carbon atoms, is preferable, and among them, a methyl group, which is the alkyl group having 1 carbon atom, is more preferable. It should be noted that, in a specific example described above, n- represents normal-form, s-represents sec-form, and t- represents tert-form.

In the case where the mono- or disulfonic acid or the salt thereof represented by the general formula [1"] is the monosulfonic acid (monosulfonic acid or salt thereof), when a carbon atom on a benzene ring bound with a sulfonic acid group is specified as 1 position, at least one moiety of p moieties of R^{1b} in the general formula [1"] is preferably the one binding to a carbon atom at 2 position, 4 position or 6 position on the benzene ring, and among them, the one binding at 4 position (p-position) is more preferable.

A specific example of the alkali metal atom represented by M in the general formula [1'] and the general formula [1 "], the alkyl group having 1 to 4 carbon atom represented by R² in the tetraalkyl ammonium (R²₄N) group represented by R²₄N group, the tetraalkyl ammonium (R²₄N) group represented by R²₄N group, n, p, and q include the same one as enumerated in the above-described general formula [1].

A specific example of the mono- or disulfonic acid or the salt thereof represented by the general formula [1'] (benzene mono- or disulfonic acid derivative) includes, for example, benzene(mono)sulfonic acid, toluenesulfonic acid (for example, o-, m- or p-toluenesulfonic acid, or the like, the same applies hereafter), ethylbenzenesulfonic acid, propylbenzenesulfonic acid, butylbenzenesulfonic acid, pentylbenzenesulfonic acid, hexylbenzenesulfonic acid, heptylbenzenesulfonic acid, octylbenzenesulfonic acid, nonylbenzenesulfonic acid, decylbenzenesulfonic acid, benzenedisulfonic acid (for example, 1,2-benzenedisulfonic acid, 1,3-benzenedisulfonic acid, 1,4-benzenedisulfonic acid, or the like), methylbenzenedisulfonic acid (for example, 5-methyl-1,3-benzenedisulfonic acid, or the like, the same applies hereafter), ethylbenzenedisulfonic acid, propylbenzenedisulfonic acid, butylbenzenedisulfonic acid, pentylbenzenedisulfonic acid, hexylbenzenedisulfonic acid, heptylbenzenedisulfonic acid, octylbenzenedisulfonic acid, nonylbenzenedisulfonic acid, decylbenzenedisulfonic acid, hydroxybenzenedisulfonic acid (for example, 4-hydroxy-1,3-benzenedisulfonic acid, 5-hydroxy-1,3-benzenedisulfonic acid, or the like), dihydroxybenzenedisulfonic acid (for example, 4,5-dihydroxy-1,3-benzenedisulfonic acid, or the like), or an alkali metal salt thereof such as a sodium salt, a potassium salt, a lithium salt; an ammonium salt; a tetraalkylammonium salt such as a tetramethylammonium salt, a tetraethylammonium salt, a tetrapropylammonium salt, a tetrabutylammonium salt. Among these benzene mono- or disulfonic acid derivatives represented by the general formula [1'], for example, benzene(mono)sulfonic acid; a benzene mono sulfonic acid derivative substituted with an alkyl group having 1 to 5 carbon atoms such as toluenesulfonic acid, ethylbenzenesulfonic acid, propylbenzenesulfonic acid, butylbenzenesulfonic acid, pentylbenzenesulfonic acid; or an alkali metal salt thereof such as a sodium salt, a potassium salt, a lithium salt; an ammonium salt; a tetraalkylammonium salt such as a tetramethylammonium salt, a tetraethylammonium salt, a tetrapropylammonium salt, a tetrabutylammonium salt are preferable, and among them, benzene(mono)sulfonic acid; a benzene monosulfonic acid derivative substituted with an alkyl group having 1 to 3 carbon atoms such as toluene sulfonic acid, ethylbenzene sulfonic acid, n-propylbenzene sulfonic acid, isopropylbenzene sulfonic acid; or an alkaline metal salt thereof such as a sodium salt, a potassium salt, a lithium salt; an ammonium salt; a tetraalkylammonium salt such as a tetramethylammonium salt, a tetraethylammonium salt, a tetrapropylammonium salt, and a tetrabutylammonium salt are preferable; and further among them, benzene(mono)sulfonic acid, toluenesulfonic acid, or alkali metal salt thereof such as a sodium salt, a potassium salt, a lithium salt; an ammonium salt; a tetraalkylammonium salt such as a tetramethylammonium salt, a tetraethylammonium salt, a tetrapropylammonium salt, a tetrabutylammonium salt; or the like are particularly preferable, and benzene(mono)sulfonic acid and p-toluene sulfonic acid are most preferable. In addition, these benzene mono- or disulfonic acid derivatives may be used alone in one kind, or may be used in combination of two or more kinds. It should be noted that, as these benzene mono- or disulfonic acid derivatives, commercially available ones may be used.

A specific example of the mono- or disulfonic acid or the salt thereof represented by the general formula [1"] (naphthalene mono- or disulfonic acid derivative) includes, for example, naphthalene(mono)sulfonic acid, methylnaphthalenesulfonic acid (for example, 2-, 3-, 4-, 5-, 6-, 7- or 8-methyl-1-naphthalenesulfonic acid, 1-, 3-, 4-, 5-, 6-, 7- or 8-methyl-2-naphthalenesulfonic acid, or the like, the same applies hereafter), ethylnaphthalenesulfonic acid, propylnaphthalenesulfonic acid, butylnaphthalenesulfonic acid, pentylnaphthalenesulfonic acid, naphthalenedisulfonic acid (for example, 1,2-naphthalenedisulfonic acid, 1,3-naphthalenedisulfonic acid, 1,4-naphthalenedisulfonic acid, 2,3-naphthalenedisulfonic acid, 1,5-naphthalenedisulfonic acid, 2,6-naphthalenedisulfonic acid, 2,7-naphthalenedisulfonic acid, or the like), hydroxynaphthalenedisulfonic acid (for example, 7-hydroxy-1,3-naphthalenedisulfonic acid, 2-hydroxy-3,6-naphthalenedisulfonic acid, or the like); or an alkali metal salt thereof such as a sodium salt, a potassium salt, a lithium salt; an ammonium salt; a tetraalkylammonium salt such as a tetramethylammonium salt, a tetraethylammonium salt, a tetrapropylammonium salt, a tetrabutylammonium salt; or the like. Among these naphthalene mono- or disulfonic acid derivatives represented by the general formula [1"], an unsubstituted naphthalenemonosulfonic acid derivative such as naphthalene(mono)sulfonic acid; or an alkali metal salt thereof such as a sodium salt, a potassium salt, a lithium salt; an ammonium salt; a tetraalkylammonium salt such as a tetramethylammonium salt, a tetraethylammonium salt, a tetrapropylammonium salt, and a tetrabutylammonium salt are preferable, and among them, naphthalene(mono)sulfonic acid is particularly preferable. In addition, these naphthalene mono- or disulfonic acid derivatives may be used alone in one kind, or may be used in combination of two or more kinds. Further, as these naphthalene mono- or disulfonic acid derivatives, the commercially available ones may be used.

Among the mono- or disulfonic acid or the salt thereof represented by the general formula [1 "], the mono- or disulfonic acid or the salt thereof represented by the general formula [1'] (benzene mono- or disulfonic acid derivative) is preferable.

There may be the case where the mono- or disulfonic acid relevant to the present invention was a mono- or disulfonic acid (what is called an acid itself), in the stage before preparing the etching solution of the present invention, but has been converted to a salt of the mono- or disulfonic acid (mono- or disulfonate salt), in the etching solution after preparation. That is, there may be the case where a salt is formed by an alkali compound of the alkali etching solution for solar cell manufacturing of the present invention or a component other than these, and the mono- or disulfonic acid relevant to the present invention, and in the present invention, the etching solution containing such a mono- or disulfonic acid is naturally included as the alkali etching solution for solar cell manufacturing of the present invention.

As for concentration of the mono- or disulfonic acid relevant to the present invention in the alkali etching solution for solar cell manufacturing of the present invention, as % by weight relative to total weight of the etching solution, the lower limit thereof is usually 0.05% by weight or higher, and preferably 0.1% by weight or higher, while the upper limit thereof is usually 30% by weight or lower, and preferably 10% by weight or lower. The case where concentration of the mono- or disulfonic acid relevant to the present invention is extremely low may provide the case where a uniform hubbly structure cannot be formed on the surface of a wafer having a silicon as a main component, while the case where concentration of the mono- or disulfonic acid relevant to the present invention is extremely high may provide the case where the mono- or disulfonic acid relevant to the present invention is deposited as a solid in the etching solution, and thus it is not desirable.

One of the characteristics of the present invention is to use the mono- or disulfonic acid relevant to the present invention, as one of the components in the etching solution. It is not necessarily true that any kind of sulfonic acid is capable of effectively forming, for example, a fine pyramid-shape (quadrangular pyramid-shape) hubbly structure on the surface of a wafer having a silicon as a main component, without any manufacturing defect, but use of the mono- or disulfonic acid relevant to the present invention is required. This reason relates to etching mechanism using the mono- or disulfonic acid relevant to the present invention, and the mechanism is considered as follows. That is, as for the mono- or disulfonic acid relevant to the present invention, the sulfonic acid group of the relevant mono- or disulfonic acid adheres on the surface of a wafer, which is a subject of etching. As a result, when an alkali compound acts on a wafer to etch the surface thereof, an aryl group of the mono- or disulfonic acid prevents the etching by the alkali compound to be described later, onto the wafer surface. In this way, a portion (point), where the mono- or disulfonic acid adhered, becomes a base point (apex), and anisotropic etching by the alkali compound proceeds to form a pyramid-shape (quadrangular pyramid-shape) hubbly structure on the wafer surface.

A specific example of (B) the alkali compound in the alkali etching solution for solar cell manufacturing of the present invention includes, for example, an alkali metal hydroxide such as sodium hydroxide, potassium hydroxide, lithium hydroxide; for example, an alkali metal carbonate such as sodium carbonate, potassium carbonate, lithium carbonate; for example, a tetraalkylammonium hydroxide such as tetramethylammonium hydroxide, tetraethylammonium hydroxide, tetrapropylammonium hydroxide, tetrabutylammonium hydroxide. Among these alkali compounds, sodium hydroxide, potassium hydroxide, sodium carbonate, potassium carbonate, and tetramethylammonium hydroxide are preferable, and among them, sodium hydroxide, and potassium hydroxide are more preferable. Particularly, sodium hydroxide and potassium hydroxide are included as the particularly preferable one, from the standpoint of possibility of efficiently etching the surface of a wafer having a silicon as a main component. In addition, as for these alkali compounds, one kind among them may be used alone, or two or more kinds may be used in combination. It should be noted that, as for these alkali compounds, commercially available ones may be used.

As for concentration of (B) the alkali compound in the alkali etching solution for solar cell manufacturing of the present invention, as % by weight relative to total weight of the etching solution, the lower limit thereof is usually 0.1% by weight or higher, preferably 0.5% by weight or higher, and more preferably 1% by weight or higher, while the upper limit thereof is usually 15% by weight or lower, preferably 10% by weight or lower, and more preferably 7% by weight or lower. The case where concentration of the alkali compound is extremely low may provide the case where an effective etching of the surface of a wafer having a silicon as a main component cannot be attained, and thus it is not desirable.

In the alkali etching solution for solar cell manufacturing of the present invention, (D) a carboxylic acid, (E) a silicic acid or a silicate, or the like may be contained, as needed, as other components than (A) the mono- or disulfonic acid or a salt thereof represented by the general formula [1], along with (B) the alkali compound, which are constituents thereof.

(D) the carboxylic acid may be added as appropriate, from the standpoint of possibility of etching the surface of wafer having a silicon as a main component in a shorter time and efficiently. Such (D) the carboxylic acid includes, for example, a saturated aliphatic monocarboxylic acid such as formic acid, acetic acid, trifluoroacetic acid, propionic acid, butyric acid, valeric acid, caproic acid, enanthic acid, caprylic acid, pelargonic acid, capric acid, lauric acid, or an isomer thereof; for example, an unsaturated aliphatic monocarboxylic acid such as acrylic acid, butenoic acid, pentenoic acid, hexenoic acid, heptenoic acid, pentadienoic acid, hexadienoic acid, heptadienoic acid, acetylene carboxylic acid, or an isomer thereof; for example, a saturated aliphatic dicarboxylic acid such as oxalic acid, malonic acid, succinic acid, glutaric acid, adipic acid, pimelic acid, cyclopentane dicarboxylic acid, cyclohexane dicarboxylic acid, or an isomer thereof; for example, an unsaturated aliphatic dicarboxylic acid such as maleic acid, fumaric acid, dimethyl maleic acid, dimethyl fumaric acid, ethyl maleic acid, ethyl fumaric acid, citraconic acid, mesaconic acid, itaconic acid, or an isomer thereof; for example, a saturated aliphatic tricarboxylic acid such as propane tricarboxylic acid, methane triacetic acid, or an isomer thereof; for example, an unsaturated aliphatic tricarboxylic acid such as aconitic acid, or an isomer thereof; for example, an aromatic monocarboxylic acid such as benzoic acid; for example, an aromatic dicarboxylic acid such as phthalic acid, isophthalic acid, terephthalic acid; for example, an aromatic tricarboxylic acid such as benzene tricarboxylic acid; for example, an aromatic hexacarboxylic acid such as mellitic acid; for example, a hydroxycarboxylic acid such as glycolic acid, lactic acid, hydracrylic acid, glyceric acid, tartronic acid, hydroxybutyric acid, malic acid, tartaric acid, citric acid, isocitric acid, gluconic acid, salicylic acid; for example, a ketocarboxylic acid such as pyruvic acid, acetoacetic acid, propionylacetic acid, levulinic acid; for example, an alkoxy carboxylic acid such as methoxy carboxylic acid, ethoxyacetic acid. For explanation convenience, a carboxylic acid having at least one hydroxyl group in a molecule is classified as a hydroxycarboxylic acid regardless of number of the carboxyl group. In addition, as for these carboxylic acids, one kind among them may be used alone, or two or more kinds may be used in combination. It should be noted that, as for these carboxylic acids, commercially available ones may be used.

As for concentration of the relevant carboxylic acid in the case of adding (D) the carboxylic acid, as % by weight relative to total weight of the etching solution, the lower limit thereof is usually 0.1% by weight or higher, while the upper limit thereof is usually 15% by weight or lower.

(E) the silicic acid or the silicate may be added, as appropriate, in view of being capable of etching the surface of a wafer having a silicon as a main component in shorter period and more effectively. Such (E) the silicic acid or the silicate includes, for example, silicic acid such as orthosilicic acid (H₄SiO₄), metasilicic acid (H₂SiO₃), metadisilic acid (H₂Si₂O₅), anhydrous silicic acid (SiO₂: silica); for example, a silicate such as sodium silicate such as sodium orthosilicate (Na₄SiO₄), sodium metasilicate (Na₂SiO₃), sodium metadisilicate (Na₂Si₂O₅), Na₂Si₄O₉; for example, potassium silicate (K₂SiO₃). In addition, as for these silicic acids or the silicates, one kind among them may be used alone, or two or more kinds may be used in combination. It should be noted that, as for these silicic acids or the silicates, commercially available ones may be used. By etching a wafer having a silicon as a main component using the alkali etching solution for solar cell manufacturing of the present invention, a silicon atom composing the wafer becomes dissolved into the etching solution, therefore, the alkali etching solution for solar cell manufacturing of the present invention becomes the one substantially containing the silicic acid or the silicate. In the present invention, the etching solution containing such the silicic acid or the silicate can also be used; therefore the alkali etching solution for solar cell manufacturing of the present invention is the etching solution which can be used repeatedly.

As for concentration of the relevant silicic acid or the relevant silicate in the case of adding (E) the silicic acid or the silicate, as % by weight relative to total weight of the etching solution, the lower limit thereof is usually 0.5% by weight or higher, while the upper limit thereof is usually 50% by weight or lower.

The alkali etching solution for solar cell manufacturing of the present invention can be prepared by dissolving (A) the mono- or disulfonic acid or the salt thereof represented by the general formula [1], (B) the alkali compound, and (D) the carboxylic acid, and/or (E) the silicic acid or the silicate, if needed, in (C) water, so as to attain the above-described % by weight. It should be noted that the preparation method is not especially limited and, for example, the one where (A) the mono- or disulfonic acid or the salt thereof represented by the general formula [1] is dissolved in suitable amount of (C) water, the one where (B) the alkali compound is dissolved in suitable amount of (C) water, the one where (D) the carboxylic acid, if needed, is dissolved in suitable amount of (C) water, along with, the one where (E) the silicic acid or the silicate, if needed, is dissolved in suitable amount of (C) water, may be mixed as appropriate; or (A) the mono- or disulfonic acid or the salt thereof represented by the general formula [1], (B) the alkali compound, along with (D) the carboxylic acid and/or (E) the silicic acid or the silicate, if needed, may be sequentially added and dissolved in (C) water in appropriate order. The alkali etching solution for solar cell manufacturing of the present invention prepared in this way is an aqueous solution, and may be subjected to filtration treatment or the like before use.

(C) Water to be used here is not especially limited, as long as it is the one not adversely influence on etching of the wafer having a silicon as a main component, and specifically includes, for example, distilled water, purified water such as deionized water, for example, ultra pure water or the like, and among them, ultra pure water is preferable. Ultra pure water is preferable in view of little contaminating the wafer after etching, because of containing almost no impurities.

As a mixing apparatus to be used in preparing the alkali etching solution for solar cell manufacturing of the present invention, for example, an agitator, a disperser or the like usually used in this field can be used. The agitator includes, for example, a mechanical stirrer, a magnetic stirrer or the like. In addition, the disperser includes, for example, a homogenizer, an ultrasonic disperser, a ball mill, a beads mill or the like.

The alkali etching solution for solar cell manufacturing of the present invention prepared in this way has a pH of an alkaline region at 25°C, and among that region, in view of being capable of etching the surface of a wafer having a silicon as a main component in short time and effectively, in the alkali etching solution for solar cell manufacturing of the present invention has preferably a pH of 12 or higher at 25°C, and among them, a pH of 14 or lower is more preferable. The case where a pH of the alkali etching solution for solar cell manufacturing of the present invention is 7 or lower (neutral or lower) causes the etching of the surface of a wafer having a silicon as a main component impossible, and thus it is not desirable.

The pH of the alkali etching solution for solar cell manufacturing of the present invention is measured using a commercial pH meter, in accordance with JIS Z8802-1984, without dilution.

In the alkali etching solution for solar cell manufacturing of the present invention, other components such as (D) the carboxylic acid, and/or (E) the silicic acid or the silicate or the like may be contained. However, to form a fine hubbly structure having a side length of the bottom face of a pyramid (quadrangular pyramid) of 7 µm or shorter in average, in forming a pyramid-shape (quadrangular pyramid -shape) hubbly structure on the surface of a wafer having a silicon as a main component, there may be the case where it is desirable that the alkali etching solution for solar cell manufacturing of the present invention does not substantially contain a component other than (A) the mono- or disulfonic acid or the salt thereof represented by the general formula [1], (B) the alkali compound along with (C) water, (the fourth component other than (A) to (C) composing the alkali etching solution for solar cell manufacturing of the present invention). It should be noted that "not substantially containing the fourth component" in this description means that "not containing the fourth component in an amount that could adversely influence on etching of the surface of a wafer having a silicon as a main component", and does not exclude even presence (contamination) of an extremely trace amount, as well as does not exclude that the silicic acid or the silicate salt generating from the wafer by etching a wafer is consequently contained in the etching solution.

It is desirable that a component of, for example, a cationic surfactant, a nonionic surfactant, an organic solvent or the like is not added in the alkali etching solution for solar cell manufacturing of the present invention. There may be the case where etching of the surface of a wafer having a silicon as a main component becomes impossible, even in the case where the cationic surfactant and/or the nonionic surfactant are added to the alkali etching solution for solar cell manufacturing of the present invention, only in such an amount as expressed by a unit of several ppms. In addition, if the organic solvent is added to the alkali etching solution for solar cell manufacturing of the present invention, the object of the present invention may not be achieved, because the hubbly structure (texture structure) cannot be formed. Accordingly, the addition of the cationic surfactant, the nonionic surfactant, and/or the organic solvent in the alkali etching solution for solar cell manufacturing of the present invention is not desirable.

The wafer having a silicon as a main component relevant to the present invention refers to the wafer having a silicon as a main component of, for example, a silicon (Si) wafer, a silicon carbide (SiC) wafer or the like, and may be an n-type silicon (Si) wafer doped with, for example, a five-valent atom such as phosphorus (P), arsenic (As), antimony (Sb) on a silicon (Si) wafer; and a p-type silicon (Si) wafer doped with, for example, three-valent atom such as boron (B), gallium (Ga) on a silicon (Si) wafer.

In the alkali etching solution for solar cell manufacturing of the present invention, the wafer having a silicon as a main component, which is an etching object, may be any of a wafer, for example, prepared by supplying the slurry mixed a coolant with an abrasive particulates (abrasive grain), to a contact part of a wire lane and a silicon ingot, and by polishing and cutting the silicon ingot, that is, a wafer prepared by what is called a free abrasive grain method; and a wafer, for example, prepared by running a wire lane fixed with abrasive particulates (abrasive grain) consisting of diamond or the like on the wire surface, then pressing the silicon ingot against this wire lane, and by polishing and cutting said silicon ingot, that is, a wafer prepared by what is called a fixed abrasive grain method. Generally, the wafer prepared by the fixed abrasive grain method is difficult to form the hubbly structure (texture structure), caused by high flatness degree of the wafer surface, however, by using the alkali etching solution for solar cell manufacturing of the present invention, even for the wafer prepared by the fixed abrasive grain method, the hubbly structure can be formed effectively at the surface of the wafer.

A wafer having a silicon as a main component relevant to the present invention may be a mirror wafer having a highly flat mirror plane, by further polishing the surface of a wafer described-above. Because a hubbly structure can be formed effectively by using the alkali etching solution for solar cell manufacturing of the present invention, even for the wafer having high flatness degree (mirror wafer or the like), the alkali etching solution for solar cell manufacturing of the present invention is the etching solution having high versatility. The alkali etching solution for solar cell manufacturing of the present invention exerts effect also in the case of performing anisotropic etching, in particular, aiming at increasing surface area.

The wafer having a silicon as a main component relevant to the present invention may be any of a monocrystal or a polycrystalline, however, in order to form a hubbly structure throughout the whole wafer surface, the monocrystal wafer is preferable, and the monocrystal wafer having the (100) plane is particularly preferable, in view of being capable of effectively preparing a substrate having high optical confinement effect (high light absorption rate and low light reflection rate) suitable for a solar cell.

The method for manufacturing the silicon-based substrate for solar cell manufacturing of the present invention is the one which comprises etching a wafer having a silicon as a main component, by using the alkali etching solution for solar cell manufacturing, containing (A) the mono- or disulfonic acid or the salt thereof represented by the above general formula [1], (B) the alkali compound, and (C) water, to form a hubbly structure at the surface of the wafer.

As a specific example of the method for manufacturing the silicon-based substrate for solar cell manufacturing of the present invention, there is included, for example, a method for heating the alkali etching solution for solar cell manufacturing of the present invention up to desired temperature as needed, and by making thus heated alkali etching solution for solar cell manufacturing of the present invention contacted with the above-described wafer having a silicon as a main component, or the like. The contact method is not especially limited, as long as it is a method usually performed in this field, for example, a dipping method, a spin (dropping) method, a spray method, or the like.

The method for manufacturing the silicon-based substrate for solar cell manufacturing of the present invention may adopt any of single wafer processing and batch processing. The single wafer processing is the one called a method generally by rotating a wafer having a silicon as a main component, one by one, and etching it by injecting the alkali etching solution for solar cell manufacturing of the present invention. The batch processing generally is the one called an etching method by dipping multiple pieces of the wafers having a silicon as a main component into the alkali etching solution for solar cell manufacturing of the present invention. In these methods, in view of manufacturing cost, the batch processing, which is capable of etching multiple pieces of the wafers at the same time, is preferable.

A temperature (etching temperature) of the heated alkali etching solution for solar cell manufacturing of the present invention, in the method for manufacturing the silicon-based substrate for solar cell manufacturing of the present invention, is not especially limited, as long as it is the temperature enabling to attain object etching on the surface of a wafer having a silicon as a main component (temperature enabling to form a hubbly structure), and, for example, the lower limit thereof is usually 50°C or higher, and preferably 60°C or higher, while the upper limit thereof is usually 100°C or lower, and preferably 95°C or lower. The case where the etching temperature is below 50°C tends to provide difficulty in progress of etching on the surface of a wafer having a silicon as a main component, while the case where etching temperature is over 100°C provides fierce change of composition ratio of the mono- or disulfonic acid relevant to the present invention, along with the alkali compound in the etching solution, due to evaporation of water in the alkali etching solution for solar cell manufacturing of the present invention in etching, which could adversely influence on etching performance, and thus it is not desirable.

Contact time (etching time) of the alkali etching solution for solar cell manufacturing of the present invention and a wafer having a silicon as a main component, in the method for manufacturing the silicon-based substrate for solar cell manufacturing of the present invention cannot be the unconditionally, because it depends on factors such as (A) to (C), which are components of the alkali etching solution for solar cell manufacturing of the present invention, or % by weight concentration of (A) to (E), etching temperature, however, in view of effective etching of the surface of a wafer having a silicon as a main component, and capability of efficiently manufacturing the silicon-based substrate for solar cell manufacturing, for example, the lower limit thereof is usually 5 minutes or longer, and preferably 10 minutes or longer, while the upper limit thereof is usually 50 minutes or shorter, and preferably 30 minutes or shorter.

As described above, the alkali etching solution for solar cell manufacturing of the present invention and the method for manufacturing the silicon-based substrate for solar cell manufacturing are the one being capable of forming a hubbly structure at the surface of a wafer having a silicon as a main component. The hubbly structure indicates a structure where a major part of the shape is pyramid-shape (quadrangular pyramid-shape), and by using the alkali etching solution for solar cell manufacturing of the present invention, the hubbly structure having, as the maximum side length of the bottom face of a pyramid (quadrangular pyramid), usually 0.1 µm or longer as the lower limit and 25 µm or shorter, preferably 7 µm or shorter, as the upper limit, can be formed. In this way, by using the alkali etching solution for solar cell manufacturing of the present invention, it is possible to prepare a substrate formed with a uniform hubbly structure throughout a whole wafer, without generating practically a flat part, and without forming practically an inferior hubbly structure.

Still more, the alkali etching solution for solar cell manufacturing of the present invention is the etching solution more superior, as compared with the conventional alkali etching solution, in the point of not requiring what is called aging processing (stabilization processing of etching rate by adding the silicic acid or the silicate into the etching solution in advance, and removing co-present residue by preliminary heating), due to stable etching rate, and also in the point of less foaming, and good breaking of foam adhered at the wafer surface, when the wafer is pulled up, thus resulting in foam disappearance from the wafer surface in a short time, and little generation of poor appearance of the wafer (water mark or the like) caused by remaining of foam at the wafer surface, or the like.

### EXAMPLES

The present invention will be explained below specifically, based on Examples and Comparative Examples, however, the present invention should not be restricted to these Examples. It should be noted that "%" in the following Examples is weight basis (w/w)%, unless specified otherwise.

### Examples 1 to 4, and Comparative Example 1: Etching tests of a wafer having a silicon as a main component using various kinds of alkali etching solution

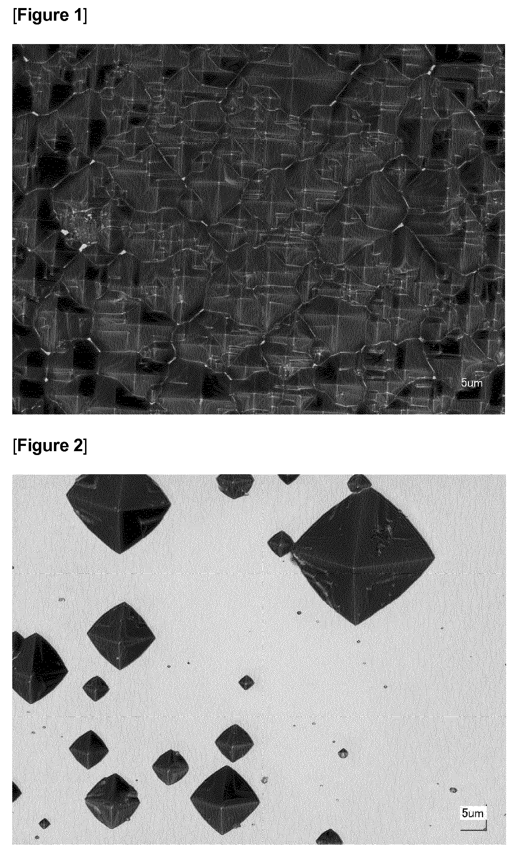

Potassium hydroxide (produced by Wako Pure Chemical Industries, Ltd.), in an amount which attains a concentration in the etching solution of % shown in Table 1, and p-toluenesulfonic acid (produced by Wako Pure Chemical Industries, Ltd.), in an amount which attains a concentration in the etching solution of % shown in Table 1, were added into ultrapure water, and then they were stirred as appropriate to prepare 5000 g of the clear etching solution having a pH of 14. Next, after heating and maintaining each etching solution at 80°C, an n-type monocrystal silicon wafer having the (100) plane (the one prepared by a free abrasive grain method: a specific resistance of 3.0 to 6.0 Ω: a size of 156 mm × 156 mm: a thickness of 200 µm), whose weight was measured beforehand, was charged into thus heated and maintained etching solution, and the wafer surface was etched by immersing it for 20 minutes, while slowly mixing with a stirrer. After that, the wafer was taken out from the solution, and subjected to over flow rinsing with ultrapure water for 20 minutes and then the wafer was naturally dried. By measuring weight of the wafer etched in this way, weight change before and after etching was calculated (decreased weight of the wafer is denoted as "matching allowance"). In addition, the wafer surface after etching was observed visually to confirm "good" or "no good" of appearance, as well as presence or absence of a hubbly structure at the wafer surface was observed using a laser microscope (VK-9700, manufactured by Keyence Corp.). It should be noted that in visual observation of appearance, it was evaluated as "good" for the case of absence of spot or irregularity at the wafer surface, and "not good" for the case of presence of staining or irregularity at the wafer surface. In addition, in observation of the hubbly structure using the laser microscope, arbitrary 5 sites on the wafer surface were observed, and it was evaluated as "good", for the case of forming a pyramid-shape (quadrangular pyramid-shape) hubbly structure at all 5 sites observed, "flatness is present" (no good), for the case of presence of a flat part even at a part thereof, and "not formed" (no good), for the case of presence of the hubbly structure (texture) having unclear ridgeline, where a pyramid (quadrangular pyramid) is not formed completely, at 50% or more of the whole. Still more, by extracting ten sites within the arbitrary one site among those observed using the laser microscope, in the order from the largest pyramid-shape (quadrangular pyramid-shape), to measure one side length of the bottom face of these pyramids (quadrangular pyramids), respectively, and the average thereof was calculated as pyramid length (quadrangular pyramid length). Composition of the etching solution, calculation result of matching amount, evaluation result of appearance, evaluation result using the laser microscope, along with calculation result of pyramid length (quadrangular pyramid length) of each Example and Comparative Example are shown in Table 1, as well as a laser microscope photograph (magnification of 3000 times) of a wafer surface after etching, obtained in Example 1 is shown in Figure 1, and a laser microscope photograph (magnification of 3000 times) of a wafer surface after etching, obtained in Comparative Example 1 is shown in Figure 2.

**[Table 1]**

| | Composition of etching solution | | | | Matching allowance (g) | Appearance evaluation | Microscope evaluation | Pyramid length (µm) |
|---|---|---|---|---|---|---|---|---|
| | Alkali compound | wt.% | Mono- or disulfonic acid | wt.% | | | | |
| Example 1 | KOH | 3% | p-toluenesulfonic acid | 4% | 1.51 | Good | Good | 8 |
| Example 2 | KOH | 3% | p-toluenesulfonic acid | 6% | 1.45 | Good | Good | 8 |
| Example 3 | KOH | 3% | p-toluenesulfonic acid | 8% | 1.38 | Good | Good | 7 |
| Example 4 | KOH | 3% | p-toluenesulfonic acid | 10% | 1.29 | Good | Good | 6 |
| | | | | | | | | |
| Comparative Example 1 | KOH | 30% | p-toluenesulfonic acid | 4% | 2.89 | No good | No good | - |

As is clear from the result of Table 1, it was clarified that the alkali etching solution for solar cell manufacturing of the present invention is the effective etching solution for forming a pyramid-shape (quadrangular pyramid-shape) hubbly structure. In addition, it was clarified that the alkali etching solution for solar cell manufacturing of the present invention is capable of not only forming an hubbly structure throughout a whole wafer, but also forming a pyramid (quadrangular pyramid) having short side length of the bottom face, that is, a small shape pyramid (quadrangular pyramid). Because such the small shape pyramid (quadrangular pyramid) has high effect of light confinement and is capable of enhancing light absorption rate (capable of suppressing light reflection rate low), by using the alkali etching solution for solar cell manufacturing of the present invention in preparing a wafer for a solar cell, a solar cell having high conversion efficiency (photoelectric conversion efficiency) can be prepared. On the other hand, as in Comparative Example 1, in the case where a wafer is etched with the etching solution, containing an alkali compound in such high concentration as 30% by weight of potassium hydroxide, it was clarified to become not capable of forming a uniform pyramid-shape (quadrangular pyramid-shape) hubbly structure, caused by too high etching speed. In addition, because the more increased concentration of the mono- or disulfonic acid relevant to the present invention provided the less matching amount, it is suggested that the aryl group of the mono- or disulfonic acid adhered at the wafer surface inhibited the etching of the alkali compound. In addition, it is clarified that the higher content of the mono- or disulfonic acid relevant to the present invention enables to form a pyramid (quadrangular pyramid) having the shorter length of one side of the bottom face, that is, the smaller shape pyramid (quadrangular pyramid).

### Examples 5 to 12: Etching tests of a wafer having a silicon as a main component under different etching condition

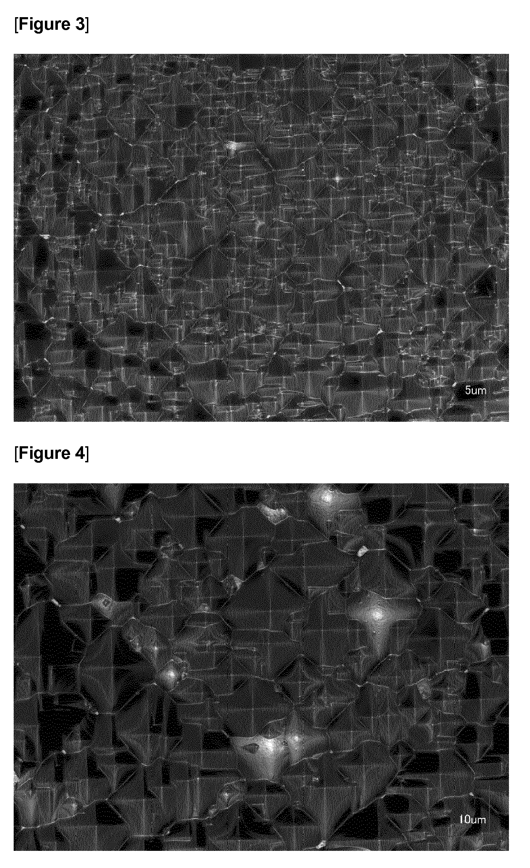

Potassium hydroxide (produced by Wako Pure Chemical Industries, Ltd.), in an amount to attain a concentration in 3% of the etching solution, and p-toluenesulfonic acid (produced by Wako Pure Chemical Industries, Ltd.), in an amount to attain a concentration in 6% of the etching solution, were added into ultrapure water, and then they were stirred as appropriate to prepare 5000 g of the clear etching solution having a pH of 14. Next, using the above-described etching solution, and under each condition shown in Table 2, similarly as in Example 1, a silicon wafer surface was etched. Rinsing and drying after etching, as well as evaluation methods of the wafer were performed also in accordance with Example 1. Etching condition, calculation result of etching amount, evaluation result of appearance, evaluation result using the laser microscope, along with calculation result of pyramid length (quadrangular pyramid length) of each Example are shown in Table 2, as well as a laser microscope photograph (magnification of 3000 times) of the wafer surface after etching, obtained in Example 7 is shown in Figure 3, and a laser microscope photograph (magnification of 3000 times) of the wafer surface after etching, obtained in Example 9 is shown in Figure 4.

**[Table 2]**

| | Etching condition | | Matching allowance (g) | Appearance evaluation | Microscope evaluation | Pyramid length (µm) |
|---|---|---|---|---|---|---|
| | Temperature of etching solution | Etching time | | | | |
| Example 5 | 80°C | 15 min. | 1.28 | Good | Good | 7 |
| Example 6 | 80°C | 40 min. | 1.93 | Good | Good | 9 |
| Example 7 | 85°C | 15 min. | 1.54 | Good | Good | 8 |
| Example 8 | 85°C | 20 min. | 1.66 | Good | Good | 9 |
| Example 9 | 90°C | 20 min. | 1.85 | Good | Good | 10 |
| Example 10 | 90°C | 15 min. | 1.70 | Good | Good | 9 |
| Example 11 | 65°C | 30 min. | 1.31 | Good | Good | 7 |
| Example 12 | 70°C | 15 min. | 1.14 | Good | Good | 7 |

As is clear from the result of Table 2, in any etching condition, it was clarified that by using the alkali etching solution for solar cell manufacturing of the present invention, it is possible not only to form a pyramid-shape (quadrangular pyramid-shape) hubbly structure throughout a whole wafer, but also to form a pyramid (quadrangular pyramid) having short side length of the bottom face, that is, a small shape pyramid (quadrangular pyramid). In addition, in the case where etching time is the same, because the higher etching temperature tends to provide the more matching allowance, and in the case of where etching temperature is the same, the longer etching time tends to provide the more matching allowance, it was clarified that, etching temperature and etching time may be set as appropriate from the above-described range, in response to objective matching allowance.

### Examples 13 to 15: Etching tests of a wafer having a silicon as a main component under different etching condition

Potassium hydroxide (produced by Wako Pure Chemical Industries, Ltd.), in an amount to attain a concentration in 3% of the etching solution, and p-toluenesulfonic acid (produced by Wako Pure Chemical Industries, Ltd.), in an amount to attain a concentration in 4% of the etching solution, were added into ultrapure water, and then they were stirred as appropriate to prepare 5000 g of the clear etching solution having a pH of 14. Next, using the above-described etching solution, and under each condition shown in Table 3, similarly as in Example 1, a silicon wafer surface was etched. Rinsing and drying after etching, as well as evaluation methods of the wafer were performed also in accordance with Example 1. Etching condition, calculation result of etching amount, evaluation result of appearance, evaluation result using the laser microscope, along with calculation result of pyramid length (quadrangular pyramid length) of each Example are shown in Table 3.

**[Table 3]**

| | Etching condition | | Matching allowance (g) | Appearance evaluation | Microscope evaluation | Pyramid length (µm) |
|---|---|---|---|---|---|---|
| | Temperature of etching solution | Eetching time | | | | |
| Example 13 | 70°C | 20 min. | 1.37 | Good | Good | 7 |
| Example 14 | 80°C | 20 min. | 1.51 | Good | Good | 8 |
| Example 15 | 90°C | 20 min. | 1.73 | Good | Good | 9 |

As is clear from the result of Table 3, it is clarified that the higher etching temperature tends to provide the more matching allowance, in the case where etching time is the same, and the lower etching temperature tends to form a pyramid (quadrangular pyramid) having the shorter side length of the bottom face, that is, the smaller shape pyramid (quadrangular pyramid).

### Examples 16 to 20: Etching tests of a wafer having a silicon as a main component under different wafer type

Potassium hydroxide (produced by Wako Pure Chemical Industries, Ltd.), in an amount to attain a concentration in 3% of the etching solution, and p-toluenesulfonic acid (produced by Wako Pure Chemical Industries, Ltd.), in an amount to attain a concentration in 4% of the etching solution, were added into ultrapure water, and then they were stirred as appropriate to prepare 5000 g of the clear etching solution having a pH of 14. Next, after heating and maintaining the above-described etching solution at 80°C, each wafer shown in Table 4 was charged into thus heated and maintained etching solution, and the wafer surface was etched by immersing it for 20 minutes, while slowly mixing with a stirrer. Rinsing and drying after etching, as well as evaluation methods of the wafer were performed in accordance with Example 1. Kind of a wafer, calculation result of matching allowance, evaluation result of appearance, evaluation result using the laser microscope, along with calculation result of pyramid length (quadrangular pyramid length) of each Example are shown in Table 4. It should be noted that the one pretreated with the alkali in the Table means a wafer, as a subject of etching, immersed for 10 minutes into a solution prepared, so as to attain a 48% of aqueous potassium hydroxide : 35% hydrogen peroxide water: water = 1 : 1 : 5, and heated and held at 70°C, and thereby the wafer surface adhered with impurities are purified.

**[Table 4]**

| | Kind of wafer | Matching allowance (g) | Appearance evaluation | Microscope evaluation | Pyramid length (µm) |
|---|---|---|---|---|---|
| Example 16 | p-type monocrystal silicon wafer (prepared by a free abrasive grain method) | 1.51 | Good | Good | 8 |
| Example 17 | p-type monocrystal silicon wafer (prepared by a fixed abrasive grain method) | 1.39 | Good | Good | 8 |
| Example 18 | p-type monocrystal silicon wafer pretreated with the alkali | 1.49 | Good | Good | 8 |
| Example 19 | n-type monocrystal silicon wafer pretreated with the alkali | 1.35 | Good | Good | 8 |
| Example 20 | Mirror wafer | 1.23 | Good | Good | 8 |

As is clear from the result of Table 4, it was clarified that the alkali etching solution for solar cell manufacturing of the present invention is also applicable to various wafers other than an n-type wafer prepared by a free abrasive grain method used in Example 1. In particular, in a conventional method, it has been difficult to form a pyramid-shape (quadrangular pyramid-shape) hubbly structure throughout a whole wafer, to a wafer prepared by a fixed abrasive grain method or mirror wafer, which tends to increase flatness degree of the wafer surface, however, it is clarified that the alkali etching solution for solar cell manufacturing of the present invention is capable of forming a fine pyramid-shape (quadrangular pyramid-shape) hubbly structure throughout the whole wafer, even to a wafer having such high flatness degree.

### Comparative Examples 2 and 3: Etching tests of a wafer having a silicon as a main component under different etching condition

Potassium hydroxide (produced by Wako Pure Chemical Industries, Ltd.), in an amount to attain a concentration in 4% of the etching solution, and isopropyl alcohol (produced by Wako Pure Chemical Industries, Ltd.), in an amount to attain a concentration in 2% of the etching solution, were added into ultrapure water, and then they were stirred as appropriate to prepare 5000 g of the clear etching solution having a pH of 14. Next, after heating and maintaining the above-described etching solution at 80°C, each wafer shown in Table 5 was charged into thus heated and maintained etching solution, and the wafer surface was etched by immersing it for 20 minutes, while slowly mixing with a stirrer. Rinsing and drying after etching, as well as evaluation methods of the wafer were performed in accordance with Example 1. Kind of a wafer, calculation result of etching amount, evaluation result of appearance, evaluation result using the laser microscope, along with calculation result of pyramid length (quadrangular pyramid length) of each Comparative Example are shown in Table 5.

**[Table 5]**

| | Kind of wafer | Matching allowance (g) | Appearance evaluation | Microscope evaluation | Pyramid length (µm) |
|---|---|---|---|---|---|
| Comparative Example 2 | p-type monocrystal silicon wafer (prepared by a fixed abrasive grain method) | 0.30 | No good | Flatness present (No good) | - |
| Comparative Example 3 | Mirror wafer | 0.24 | No good | Not formed (No good) | - |

As is clear from the result of Table 5, it was clarified that in the case of using an the alkali etching solution containing isopropyl alcohol, which has been used conventionally, a pyramid-shape (quadrangular pyramid-shape) hubbly structure cannot form throughout a whole wafer, on the surface of a wafer prepared by a fixed abrasive grain method or mirror wafer, which tends to increase flatness degree of the wafer surface, or a large number of pyramids (quadrangular pyramids) having an unclear ridgeline are generated.

### Examples 21 and 22: Etching tests of a wafer having a silicon as a main component under different composition of the etching solution

Potassium hydroxide (produced by Wako Pure Chemical Industries, Ltd.), in an amount to attain a concentration in 3% of the etching solution, p-toluenesulfonic acid (produced by Wako Pure Chemical Industries, Ltd.), in an amount to attain a concentration in 6% of the etching solution, and sodium silicate in an amount to attain a concentration in 2% or 4% of the etching solution, were added into ultrapure water, and then they were stirred as appropriate to prepare 5000 g of the clear etching solution having a pH of 14. Next, after heating and maintaining each etching solution at 80°C, a similar silicon wafer as in Example 1 was charged into thus heated and maintained etching solution, and the wafer surface was etched by immersing it for 20 minutes, while slowly mixing with a stirrer. Rinsing and drying of the silicon wafer after etching, as well as evaluation methods of the wafer were performed in accordance with Example 1. Composition of the etching solution is shown in Table 6, as well as calculation result of etching amount, evaluation result of appearance, evaluation result using the laser microscope, along with calculation result of pyramid length (quadrangular pyramid length) of each Example are shown in Table 7.

**[Table 6]**

| | Composition of etching solution | | | | | |
|---|---|---|---|---|---|---|
| | Alkali compound | wt.% | Mono- or disulfonic acid | wt.% | Silicate | wt.% |
| Example 21 | KOH | 3% | p-toluenesulfonic acid | 6% | Sodium silicate | 2% |
| Example 22 | KOH | 3% | p-toluenesulfonic acid | 6% | Sodium silicate | 4% |

**[Table 7]**

| | Matching allowance (g) | Appearance evaluation | Microscope evaluation | Pyramid length (µm) |
|---|---|---|---|---|
| Example 21 | 1.40 | Good | Good | 8 |
| Example 22 | 1.42 | Good | Good | 8 |

As is clear from the result of Table 6 and Table 7, it was clarified that, even the etching solution containing a component (the forth component), for example, a silicate or the like, other than the mono- or disulfonic acid relevant to the present invention as well as the alkali compound, which are constituents of the present invention, can form a pyramid-shape (quadrangular pyramid-shape) hubbly structure throughout a whole wafer, on the surface of a wafer having a silicon as a main component.

### Example 23, and Comparative Examples 4 and 5: Foaming tests of the etching solution using various alkali etching solution.

Potassium hydroxide (produced by Wako Pure Chemical Industries, Ltd.), in an amount to attain a concentration in 4% of the etching solution, and various kinds of sulfonic acids or salts thereof (all produced by Wako Pure Chemical Industries, Ltd.) shown in Table 8, in an amount to attain a concentration in 1% of the etching solution of 1%, were added into ultrapure water, and then they were stirred as appropriate to prepare 20 mL of the clear etching solution having a pH of 14. Next, each etching solution was shaken at room temperature (20°C) for 1 minute, and volume of foam derived from the etching solution and generated after shaking was measured, as well as foam disappearance degree was observed. It should be noted that foam disappearance degree was judged " 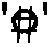 " when foam generated after shaking has disappeared almost after 1 minute, and "×" when foam was remained almost. Composition of the etching solution, measurement result of foam volume derived from each etching, along with disappearance degree of foam derived from each etching solution of each Example and Comparative Examples are shown in Table 8.

**[Table 8]**

| | Composition of etching solution | | | | Volume of foam (cm³) | Foam disappearance degree |
|---|---|---|---|---|---|---|
| | Alkali compound | wt.% | Mono- or disulfonic acid (salt) | wt.% | | |
| Example 23 | KOH | 4% | p-toluenesulfonic acid | 1% | 2 | ○ |
| | | | | | | |
| Comparative Example 4 | KOH | 4% | sodium n-octanesulfonate | 1% | 20 | × |
| Comparative Example 5 | KOH | 4% | sodium n-decanesulfonate | 1% | 30 | × |

As is clear from the result of Table 8, it was clarified that the etching solution containing the mono- or disulfonic acid relevant to the present invention, as the mono- or disulfonic acid (salt), provides foam disappearance from the wafer surface in a shorter time, because of less foaming and good breaking of foam adhered at the wafer surface in pulling up the wafer. That is, in etching, because foaming of the etching solution is generated by hydrogen generated from the wafer surface, the surface of the etching solution is covered with foam. Accordingly, foam covering the surface of the etching solution, in pulling up the wafer, adheres to the wafer surface, however, because the etching solution containing the mono- or disulfonic acid relevant to the present invention provides less foaming, and foam disappears from the wafer surface in a shorter time, as a result, in the case that a wafer was etched with the alkali etching solution for solar cell manufacturing of the present invention, it is suggested that foam does not remain on the surface of the wafer pulled up, and appearance defect of the wafer is hard to occur. On the other hand, because the etching solution containing an alkylsulfonic acid (or a salt thereof) such as sodium n-octanesulfonate or sodium n-decanesulfonate, as the mono- or disulfonic acid, provides fierce foaming, and poor breaking of foam adhered at the wafer surface when pulled up, it is clarified that foam remains on the wafer surface. That is, it is as described above that foam covering the surface of the wafer adheres to the surface of the wafer in pulling up the wafer, however, because the etching solution containing the alkylsulfonic acid (or the salt thereof) provides fierce foaming and foam does not practically disappear from the wafer surface, as a result, it is suggested that, in the case that wafer is etched with the etching solution containing the alkylsulfonic acid (or the salt thereof), foam remains on the surface of the wafer in pulling up, and water mark is generated, which tends to generate appearance defect of a wafer,.

As is clear from the above results, it was clarified that the alkali etching solution for solar cell manufacturing of the present invention is the superior etching solution which can form a pyramid-shape (quadrangular pyramid-shape) hubbly structure throughout a whole wafer having a silicon as a main component. In addition, because the alkali etching solution for solar cell manufacturing of the present invention can effectively form a pyramid-shape (quadrangular pyramid-shape) hubbly structure, even for a wafer prepared by a fixed abrasive grain method or mirror wafer, which has been said difficult to form a uniform hubbly structure throughout a whole wafer by a conventional method, it was clarified that the alkali etching solution for solar cell manufacturing of the present invention is the superior etching solution applicable to various wafers. Still more, because application of the alkali etching solution for solar cell manufacturing of the present invention to a monocrystal wafer is capable of forming a fine pyramid-shape (quadrangular pyramid-shape) hubbly structure suitable for a texture structure on the wafer surface, it was also clarified that the alkali etching solution for solar cell manufacturing of the present invention is the preferable etching solution to produce a wafer suitable for a solar cell superior in conversion efficiency (photoelectric conversion efficiency). Furthermore, because of less foaming, and good breaking of foam adhered at the wafer surface, when the wafer is pulled up, it was also clarified that the alkali etching solution for solar cell manufacturing of the present invention is the etching solution providing disappearance from the wafer surface in a short time, and being difficult to generate appearance defect of the wafer caused by remaining of foam on the wafer surface.

### INDUSTRIAL APPLICABILITY

The alkali etching solution for solar cell manufacturing of the present invention is the etching solution which can form a hubbly structure throughout a whole wafer having a silicon as a main component. In more detail, the alkali etching solution for solar cell manufacturing of the present invention is the one capable of forming a fine pyramid-shape (quadrangular pyramid-shape) hubbly structure on the wafer surface, and can be used in manufacturing, in particular, a silicon-based substrate suitable for a solar cell.

A method for manufacturing the silicon-based substrate for solar cell manufacturing of the present invention is an effective method to form a pyramid-shape (quadrangular pyramid-shape) hubbly structure on the surface of a wafer having a silicon as a main component, in particular, a preferable method for manufacturing the silicon-based substrate for a solar cell superior in conversion efficiency (photoelectric conversion efficiency).

## Claims

1. An alkali etching solution for solar cell manufacturing, comprising (A) a mono- or disulfonic acid or a salt thereof represented by the general formula [1], (B) an alkali compound, and (C) water: 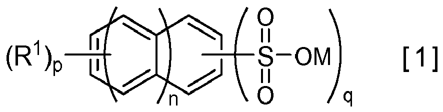 {wherein p moieties of R¹ each independently represents a hydrogen atom, a hydroxyl group or an alkyl group having 1 to 10 carbon atoms; q moieties of M each independently represents a hydrogen atom, an alkali metal atom, an ammonium (NH₄) group or a tetraalkylammonium (R²₄N) group (wherein R² represents an alkyl group having 1 to 4 carbon atoms); n represents 0 or 1; and p and q each independently represents 1 or 2}.

2. The etching solution according to claim 1, wherein concentration of the above-described (A) mono- or disulfonic acid or a salt thereof represented by the general formula [1] is 0.05% by weight to 30% by weight.

3. The etching solution according to claim 1, wherein concentration of the above-described (B) alkali compound is 0.1% by weight to 15% by weight.

4. The etching solution according to claim 1, wherein the above-described (A) mono- or disulfonic acid or a salt thereof represented by the general formula [1] is a mono- or disulfonic acid or a salt thereof represented by the general formula [1']: 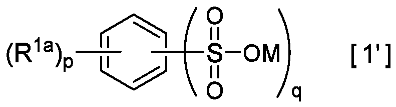 (wherein p moieties of R^{1a} each independently represents a hydrogen atom, a hydroxyl group or an alkyl group having 1 to 10 carbon atoms; and M, p and q are the same as described above).

5. The etching solution according to claim 1, wherein the above-described (A) mono- or disulfonic acid or a salt thereof represented by the general formula [1] is toluenesulfonic acid or a salt thereof.

6. The etching solution according to claim 1, wherein the above-described (B) alkali compound is an alkali metal hydroxide, an alkali metal carbonate or tetraalkylammonium hydroxide.

7. The etching solution according to claim 1, wherein the above-described (B) alkali compound is sodium hydroxide, potassium hydroxide, sodium carbonate, potassium carbonate or tetramethylammonium hydroxide.

8. The etching solution according to claim 1, wherein the above-described (B) alkali compound is sodium hydroxide or potassium hydroxide.

9. A method for manufacturing a silicon-based substrate for solar cell manufacturing, which comprises etching a wafer having a silicon as a main component, by using the etching solution according to claim 1, to form a hubbly structure at the surface of the wafer.

10. The method for manufacturing the silicon-based substrate according to claim 9, wherein the above-described wafer is etched using the above-described etching solution at 50°C to 100°C.

11. The method for manufacturing the silicon-based substrate according to claim 9, wherein the above-described hubbly structure is the one having a pyramid shape, and the maximum side length of the bottom face thereof is 0.1 µm to 25 µm.

12. The method for manufacturing the silicon-based substrate according to claim 9, wherein the above-described wafer is a monocrystal wafer.
